# EUROPÄISCHE PATENTANMELDUNG

(11) **EP 3 960 047 A1**
(43) Veröffentlichungstag der Anmeldung: **02.03.2022**
(21) Anmeldenummer: 21193329.6
(22) Anmeldetag: 26.08.2021
(51) Int. Cl.: A47K 3/30, A61B 5/00, A47K 3/26

(54) **TECHNIKBOX UND WC-SITZGARNITUR**

(30) Priorität: 28.08.2020 DE 102020122540; 01.06.2021 DE 102021114175
(71) Anmelder: Hamberger Industriewerke GmbH, 83071 Stephanskirchen (DE)
(72) Erfinder:
(74) Vertreter: Winter, Brandl - Partnerschaft mbB

(57) **Zusammenfassung**

Offenbart ist eine WC-Sitzgarnitur mit einer Technikbox, in der Funktionskomponenten und eine Steuerelektronik der WC-Sitzgarnitur aufgenommen sind. Die Ansteuerung der WC-Sitzgarnitur erfolgt vorzugsweise über ein externes Bediengerät.

## Beschreibung

Die Erfindung betrifft eine Technikbox gemäß dem Oberbegriff des Patentanspruches 1 und eine mit einer derartigen Technikbox ausgeführte WC-Sitzgarnitur.

Seit einiger Zeit besteht ein Trend dahingehend, möglichst viele Funktionen in eine WC-Sitzgarnitur zu integrieren. So wird beispielsweise in der auf die Anmelderin zurückgehenden DE 10 2016 108 379 A1 vorgeschlagen, eine Sensorik zur Erfassung gesundheitsspezifischer Parameter (Vitalparameter) in eine WC-Sitzgarnitur aufzunehmen und die Auswertung und das Auslesen der Parameter über eine Dockingstation durchzuführen.

In der weiteren Patentanmeldung der Anmelderin DE 10 2018 106 727 A1 wird eine WC-Sitzgarnitur beschrieben, in die eine Sitzheizung, ein Temperatursensor, ein Anwesenheitssensor und eine Steuereinheit zur Ansteuerung/Auswertung der vorgenannten Komponenten integriert ist.

In der WO 2016/020409 A1 ist eine WC-Sitzgarnitur offenbart, bei der sitzseitig elektrische Verbraucher, beispielsweise eine Sitzheizung und ein Sensor integriert sind. Die Stromversorgung dieser Verbraucher erfolgt über eine in einer Technikbox integrierte Steuereinheit, wobei die Technikbox auf die Keramik aufgesetzt ist und vorzugsweise der Sitz und der Deckel an der Technikbox gelagert sind. Diese ist somit Teil der WC-Sitzgarnitur.

In der US 2018/0220859A1 ist eine WC-Sitzgarnitur offenbart, bei der in einer Art Technikbox ein Beleuchtungsmodul integriert ist, über das das Innere der Keramik ausgeleuchtet wird.

In der WO 2020/132293 A1 ist eine WC-Sitzgarnitur mit einer Waschvorrichtung offenbart, wobei innerhalb einer Technikbox unterschiedliche Flüssigkeiten gespeichert sind, die je nach Ansteuerung der WC-Sitzgarnitur über Dispenser abgegeben werden können. Die Ansteuerung dieser Dispenser kann über eine Art Fernbedienung erfolgen.

In den beiden auf die Anmelderin zurückgehenden Druckschriften DE 10 2019 110 287 A1 und DE 10 2019 134 969 A1 sind WC-Sitzgarnituren beschrieben, bei denen in einer Technikbox Elemente zur kontaktlosen Daten- und Energieübertragung angeordnet sind, die mit Funktionskomponenten des WC-Sitzes der WC-Sitzgarnitur im Hinblick auf die Energie- bzw. Datenübertragung in Wirkverbindung stehen. Die Sitzheizung ist dabei bei einem Ausführungsbeispiel auf einem Träger angeordnet, der auch Teile der die kontaktlose Nahfeldübertragung ermöglichenden Funktionskomponenten und die zugehörige Steuerungselektronik enthält. Diese WC-Sitzgarnituren können mit einem externen Bedien- und Anzeigeelement ausgeführt sein, um Funktionen zu steuern oder Informationen anzuzeigen.

In der Druckschrift DE 20 2011 108 948 U1 ist eine ähnliche WC-Sitzgarnitur offenbart, bei der die Funktionskomponenten und die Steuerungselektronik ebenfalls in einer Art Technikbox angeordnet sind, wobei die Energieversorgung von in dem WC-Sitz aufgenommenen Verbrauchern, beispielsweise einer Sitzheizung, ebenfalls kontaktlos erfolgt.

Die Betätigung der in der Technikbox oder in der WC-Sitzgarnitur integrierten Funktionskomponenten erfolgt üblicherweise durch Tasten, die an der WC-Sitzgarnitur ausgebildet sind und über die Standardfunktionen, beispielsweise das Einschalten einer Sitzheizung, das Anheben oder Absenken eines WC-Deckels, das Ein- oder Ausschalten einer Beleuchtung, etc. gesteuert werden können. Derartige kabelgebundene Lösungen sind jedoch sehr umständlich in der Handhabung und genügen auch nicht den üblichen Anforderungen an die Ästhetik.

Demgegenüber liegt der Erfindung die Aufgabe zugrunde, eine Technikbox und eine mit einer derartigen Technikbox ausgeführte WC-Sitzgarnitur zu schaffen, bei der der Bedienkomfort verbessert ist.

Diese Aufgabe wird im Hinblick auf die Technikbox durch die Merkmale des Patentanspruches 1 und im Hinblick auf die WC-Sitzgarnitur durch die Merkmale des nebengeordneten Patentanspruches 11 gelöst.

Vorteilhafte Weiterbildungen der Erfindung sind Gegenstand der Unteransprüche.

Erfindungsgemäß ist die Technikbox einer WC-Sitzgarnitur zugeordnet, die über Schwenkgelenke mit der Technikbox verbunden ist und die ihrerseits über Befestigungsmittel an einer Keramik festlegbar ist. In einem Gehäuse der Technikbox sind Funktionskomponenten und eine Steuerungselektronik der WC-Sitzgarnitur angeordnet. Erfindungsgemäß hat die Technikbox ein mehreren Funktionskomponenten und/oder der Steuerungselektronik und/oder einer externen Informationsquelle zugeordnetes, zentrales Kommunikationsmodul, dem ein externes Bedienelement, vorzugsweise ein Handheld, zugeordnet ist, das ausgelegt ist, auf der Basis einer kontaktlosen Kommunikation mit dem in die Technikbox integrierten Kommunikationsmodul die Funktionskomponenten und/oder die Steuerungselektronik der WC-Sitzgarnitur anzusteuern oder externe Informationen wiederzugeben oder eine Datenverarbeitung durchzuführen.

Durch dieses externe Bedienelement können herkömmliche Tasten an der WC-Sitzgarnitur entfallen, da die Bedienung sehr komfortabel ohne direkten körperlichen Kontakt mit der WC-Sitzgarnitur zur Einstellung unterschiedlicher Funktionen, beispielsweise dem Einschalten einer Sitzheizung oder eines Lüfters, durchgeführt werden kann.

Die kontaktlose Signal-/Datenübertragung kann beispielsweise mittels WLAN, Wifi, Bluetooth, ZigBee, NFC, Wibree, BLE, IrDA, WIS oder Funk erfolgen. Prinzipiell sind natürlich auch andere kontaktlose Daten-/Signalübertragungsverfahren anwendbar.

Das Kommunikationsmodul und das Bedienelement sind entsprechend dieses Datenübertragungsverfahrens ausgelegt.

Die Bedienung ist besonders komfortabel, wenn das Bedienelement eine Bedienfläche mit mehreren, vorzugsweise touch-sensitiven Funktionsfeldern hat. Ein derartiges Konzept ermöglicht es, unterschiedlichste Funktionen über ein sehr kompaktes externes Bedienelement anzusteuern.

Dabei kann die Bedienfläche mit einem Gestenfeld, einem Scrollfeld, einem Display, einem OK-Button, einem Return-Button oder dergleichen ausgeführt sein, die jeweils ausgelegt sind, durch vorbestimmte Betätigungsmuster dieser Bedienfläche zugeordnete Funktionen der WC-Sitzgarnitur anzusteuern oder auszulösen.

Mit anderen Worten gesagt, durch bestimmte Kombinationen der oben genannten Bedienfeldbereiche können zugeordnete Funktionen der WC-Sitzgarnitur angesteuert werden, so dass auch bei einer vergleichsweise geringen Anzahl von Bedienfeldern eine Vielzahl von Funktionen ansteuerbar ist.

Das Gestenfeld kann bei einem Ausführungsbeispiel der Erfindung so ausgelegt sein, dass Funktionen der WC-Sitzgarnitur durch Verschieben eines oder mehrerer Finger entlang des Gestenfelds nach einem vorbestimmten Muster ausgewählt werden.

Diese Muster können beispielsweise lineare Bewegungen in der Horizontalen/Vertikalen oder aber auch kreisförmige Bewegungen im oder entgegen dem Uhrzeigersinn sein. Prinzipiell können auch Buchstaben oder Buchstabenfolgen durch Überstreichen im Gestenfeld eingegeben werden.

Bei einem besonders kompakten externen Bediengerät können unterhalb einer vergleichsweise großen Gestenfläche etwa mittig ein Display und beidseitig davon OK- und Return-Buttons einerseits und eine Scrollfläche zur Anzeige unterschiedlicher Funktionen im Display andererseits angeordnet sein.

Bei einem bevorzugten Ausführungsbeispiel der Erfindung ist das Kommunikationsmodul ausgelegt, Wetterdaten oder sonstige Informationen zu erfassen und über das Bediengerät an den Nutzer/die Nutzerin weiterzugeben.

Das Bediengerät ist gemäß einer erfindungsgemäßen Weiterbildung nicht nur zum Auslösen/Eingeben von Funktionen ausgelegt, sondern die Software kann auch so ausgeführt sein, dass über die Sensorik erfasste Parameter, beispielsweise Vitalparameter, ausgewertet werden und die Ergebnisse der Auswertung dann zur Anzeige gebracht werden, um den Nutzer/die Nutzerin entsprechend zu informieren.

Bei einem Ausführungsbeispiel der Erfindung ist das Bediengerät mit einem aufladbaren Akku ausgeführt.

Die erfindungsgemäße WC-Sitzgarnitur ist mit einer derartigen Technikbox ausgeführt.

Bevorzugte Ausführungsbeispiele der Erfindung werden im Folgenden anhand schematischer Zeichnungen näher erläutert. Es zeigen:
Figur 1 eine vereinfachte Gesamtdarstellung einer erfindungsgemäßen WC-Sitzgarnitur;
Figur 2 eine Seitenansicht der WC-Sitzgarnitur gemäß Figur 1;
Figur 3 eine Rückansicht der WC-Sitzgarnitur gemäß den Figuren 1 und 2;
Figur 4 eine dreidimensionale Vorderansicht einer Technikbox der WC-Sitzgarnitur gemäß den Figuren 1 bis 3;
Figur 5 eine Rückansicht der Technikbox gemäß Figur 4;
Figur 6 eine Unteransicht der Technikbox gemäß den Figuren 4 und 5;
Figur 7 die Technikbox gemäß den Figuren 4 und 5 mit Schwenkgelenken;
Figur 8, 9 Ansichten eines Gehäusebodens der Technikbox;
Figur 10, 11 Ansichten eines Gehäuseträgers der Technikbox;
Figur 12, 13 Ansichten einer Außenschale der Technikbox;
Figur 14 eine Innenansicht des Gehäuseträgers mit integrierten Funktionskomponenten und Steuerplatinen;
Figur 15 eine Ansicht des Gehäusebodens mi einem Schwenkantrieb und einem Dämpfer;
Figur 16 eine weitere Darstellung des Gehäusebodens mit Komponenten eines Deodorizers;
Figuren 17a, 17b Ansichten einer Schublade des Deodorizers gemäß Figur 16;
Figuren 18a, 18b Ansichten der Schublade gemäß den Figuren 17a, 17b mit einer darin aufgenommenen Kassette/Kapsel;
Figur 19 einen Schnitt entlang der Linie A-A durch die Technikbox;
Figur 20 ein Ausführungsbeispiel einer WC-Sitzgarnitur mit induktiver Energieversorgung bei geöffneter WC-Sitzgarnitur;
Figur 21 die WC-Sitzgarnitur gemäß Figur 20 im geschlossenen Zustand;
Figur 22 eine Prinzipskizze zur Erläuterung einer Fernbedienung der erfindungsgemäßen WC-Sitzgarnitur und
Figur 23 ein Handheld zur Fernbedienung der erfindungsgemäßen WC-Sitzgarnitur.

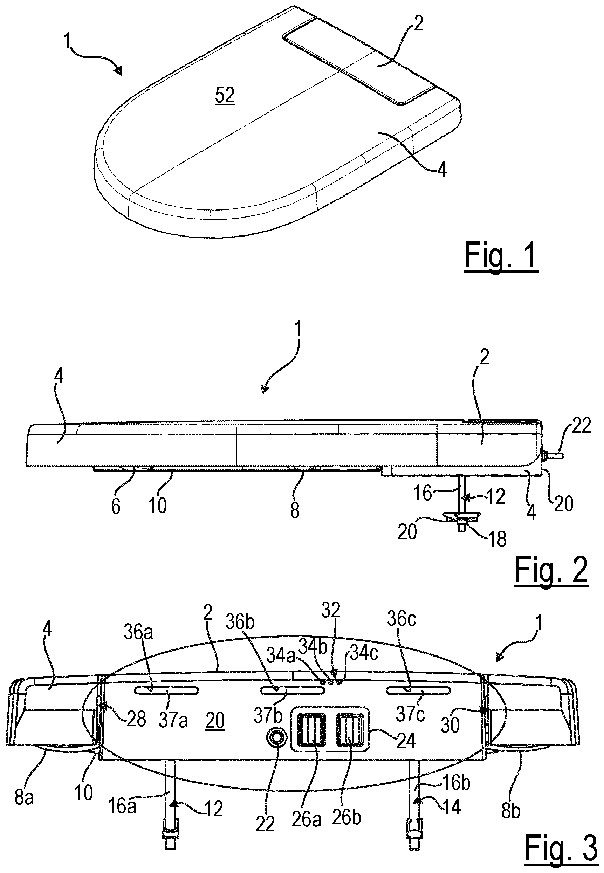

Figur 1 zeigt eine dreidimensionale Ansicht eines Ausführungsbeispiels einer erfindungsgemäßen WC-Sitzgarnitur 1, die mit einer Technikbox 2 ausgeführt ist, an der ein WC-Deckel 4 und ein WC-Sitz 10 (siehe Figur 2) schwenkbar gehalten sind, wobei letzterer in der Darstellung gemäß Figur 1 von dem WC-Deckel 4 überdeckt ist. Die WC-Sitzgarnitur 1 ist bei dem in Figur 1 dargestellten Ausführungsbeispiel in einem Flat-Design ausgeführt, wobei die Technikbox 2 bündig in den WC-Deckel 4 (im geschlossenen Zustand) integriert ist.

In der Seitenansicht gemäß Figur 2 sind Puffer 6, 8 dargestellt, mit denen der WC-Sitz 10, von dem man in Figur 2 lediglich einen Innenrand sieht, auf der Keramik (nicht dargestellt) abgestützt ist. Wie im Folgenden noch näher erläutert wird, sind der WC-Deckel 4 und der WC-Sitz 10 schwenkbar über Schwenkgelenkanordnungen mit der Technikbox 2 verbunden. Diese ist über Befestigungsmittel 12, 14 (siehe Figur 3) an der Keramik befestigt. Die Befestigungsmittel 12, 14 und die entsprechenden Befestigungselemente der Technikbox 2 sind als Take-Off-System ausgebildet, so dass die WC-Sitzgarnitur 1 auf einfache Weise zum Reinigen oder Austausch von der Keramik abgenommen werden kann. Beim dargestellten Ausführungsbeispiel sind die Befestigungsmittel 12, 14 jeweils mit einem Gewindebolzen 16 ausgeführt, der in eine Bohrung der Keramik eingesetzt wird, wobei die Lagefixierung über eine Mutter 18 erfolgt, die über eine verschwenkbare Handhabe 21 angezogen werden kann.

Aus der in Figur 2 rechts dargestellten Rückwandung 20 der Technikbox 2 tritt eine Leitung 22 aus, über die die Technikbox 2 an eine Stromversorgung angeschlossen ist. Diese Leitung 22 kann auch als Signalleitung ausgebildet sein, um über Sensoren erfasste Daten an eine Auswerteeinheit zu übergeben. Natürlich ist auch eine drahtlose Datenübertragung möglich.

In Figur 3 ist die WC-Sitzgarnitur 1 mit der Technikbox 2, von der in Figur 3 lediglich die Rückwandung 20 sichtbar ist, und mit dem WC-Deckel 4 sowie dem WC-Sitz 10 und den Puffern 8a, 8b (die vorderen Puffer 6a, 6b sind überdeckt) zu sehen. Aus der Rückwandung 20 tritt, wie erläutert, die Leitung 22 aus. In Figur 3 neben dieser Leitung 22 ist eine Schublade 24 mit Griffmulden 26a, 26b angeordnet, die aus der Rückwandung 20 zum Betrachter hin (Figur 3) herausziehbar ist und die ein Duftmittel eines Deodorizers trägt, der im Folgenden noch näher beschrieben wird.

In der Darstellung gemäß Figur 3 sieht man auch zwei abschnittsweise Schwenkgelenke 28, 30, die an der Technikbox 2 gehalten sind und über die der WC-Deckel 4 schwenkbar gelagert ist. Auf den Aufbau dieser Schwenkgelenke 28, 30, die einerseits durch eine von einem Antriebsmotor angetriebene Gelenkwelle und einen Dämpfer und andererseits als Schwenkzapfen ausgebildet sein können, wird in der Folge noch näher eingegangen.

In Figur 3 oberhalb der Schublade 24 ist ein Lufteinlass 32 des Deodorizers angeordnet, der in der Rückwandung 20 mündet und beim dargestellten Ausführungsbeispiel mit drei Einlassausnehmungen 34a, 34b, 34c ausgebildet ist.

In der Rückwandung 20 sind beim dargestellten Ausführungsbeispiel noch drei Licht-Austrittsöffnungen 36a, 36b, 36c ausgebildet, durch die hindurch von einem Lichtmodul 190 abgegebenes Licht nach außen hin abstrahlt, so dass die WC-Sitzgarnitur 1 indirekt beleuchtet ist. Dabei ist in diese Austrittsöffnungen 36a, 36b, 36c jeweils ein Licht-Austrittselement ausgebildet, das beim dargestellten Ausführungsbeispiel als Glaseinsatz 37a, 37b, 37c ausgebildet ist, der passgenau in die Austrittsöffnungen 36a, 36b, 36c eingesetzt ist und somit als eine Art Austrittsoptik wirkt. Auch dieses Lichtmodul 190 wird im Folgenden erläutert.

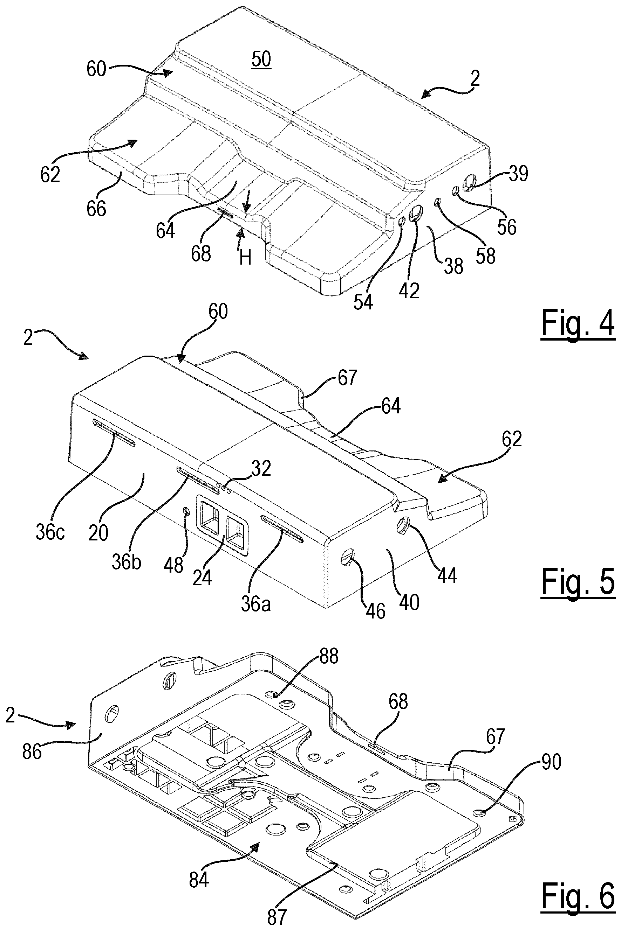

Die Figuren 4 und 5 zeigen Ansichten der in Figur 1 dargestellten Technikbox 2 der WC-Sitzgarnitur 1, wobei die vorbeschriebenen Schwenkgelenke 28, 30, die an dieser Technikbox 2 gehalten sind und sonstige Ein- und Ausbauten nicht dargestellt sind.

Figur 4 zeigt eine Vorderansicht der Technikbox 2, die im montierten Zustand zu dem von der Keramik begrenzten Aufnahmeraum ausgerichtet ist. An der in Figur 4 rechts dargestellten Seitenwandung 38 sind Durchbrüche 39, 42 ausgebildet, durch die hindurch sich die Schwenkgelenke 28, 30 zum WC-Deckel 4 bzw. zum WC-Sitz 10 hin erstrecken. An der in Figur 5 dargestellten anderen Seitenwandung 40 der Technikbox 2 sind weitere Durchbrüche 44, 46 für Schwenkzapfen der Schwenkgelenke 28, 30 vorgesehen. In der Rückwandung 20 der Technikbox 2 sind entsprechend die drei Austrittsöffnungen 36a, 36b, 36c des Lichtmoduls, die Schublade 24 und der Lufteinlass 32 des Deodorizers sowie eine Durchführung 48 für die Leitung 22 ausgebildet. Wie vorstehend erläutert, ist eine Deckfläche 50 der Technikbox 2 bündig mit der in Figur 1 sichtbaren Großfläche 52 des WC-Deckels 4 angeordnet, wobei beide gemeinsam in etwa eine horizontale Ebene ausbilden. Im Bereich neben den Durchbrüchen 39, 42 der Seitenwandung 39 sind noch Ausnehmungen 54, 56 ausgebildet, durch die hindurch sich Zentrierstifte der Schwenkgelenke 28, 30 erstrecken, die in Führungsnuten der Kloben des WC-Deckels 4 und des WC-Sitzes 10 eintauchen und deren Schwenkbewegung stützen/führen. Zwischen der Ausnehmung 56 und dem Durchbruch 42 ist eine weitere Durchführung 68 für Kabel oder dergleichen vorgesehen.

Die Technikbox 2 ist zur Keramik hin zurückgestuft, wobei sich an die Deckfläche 50 eine Zwischenstufe 60 anschließt, die ihrerseits in eine flache Austrittsstufe 62 übergeht, wobei diese jeweils leicht schräg zur Horizontalen angestellt sind. Die Austrittsstufe 62 ist im mittleren Bereich mit einer Einschnürung 64 ausgeführt, durch die die Höhe H einer Stirnfläche 66 der Technikbox 2 und damit auch der Austrittsstufe 62 rinnenförmig verringert wird. Im Bereich dieser Einschnürung 64 mündet in der Stirnfläche 66 ein Luftauslass 68 des Deodorizers. Dementsprechend ist über die Deckfläche der Einschnürung 64 der Strömungsquerschnitt des Deodorizer-Kanals hin zum Luftauslass 68 verringert - auf diese Strömungsführung wird in der Folge noch näher eingegangen. Im Bereich dieser rinnenförmigen Einschnürung 64 ist die Stirnfläche 66 mit einer etwa U-förmigen Einwölbung 67 ausgeführt, in der der Luftauslass 68 mündet.

Figur 6 zeigt eine Unteransicht der Technikbox 2, in der ein Gehäuseboden 84 sichtbar ist, der die Technikbox 2 nach unten, zur Keramik hin im wesentlichen luft- und flüssigkeitsdicht schließt. In der Darstellung gemäß Figur 6 ist zwar eine Reihe von Durchbrüchen dargestellt, die jedoch durch entsprechende innen liegende Komponenten abgedeckt sind. Des Weiteren sichtbar sind Einprägungen, die ebenfalls entsprechend der in der Technikbox 2 aufgenommenen Funktionskomponenten ausgeführt sind und über die die Verwindungssteifigkeit des Gehäusebodens 84 optimiert ist. Eine dieser Einprägungen ist beispielhaft in Figur 6 mit dem Bezugszeichen 87 versehen.

Wie vorstehend erläutert, ist die WC-Sitzgarnitur 1 mit der Technikbox 2 über ein Take-Off-System lösbar an der Keramik befestigt. Dabei tauchen die beiden Befestigungsmittel 12, 14, genauer gesagt deren Gewindebolzen 16a, 16b (siehe Figur 3) in zwei Aufnahmen 88, 90 des Gehäusebodens 84 ein und sind dort so über das Take-Off-System lösbar verankert, dass sie nach unten, zur Keramik hin auskragen.

Weitere Einzelheiten dieses Gehäusebodens 84 und der sonstigen Komponenten der Technikbox 2 werden im Folgenden erläutert.

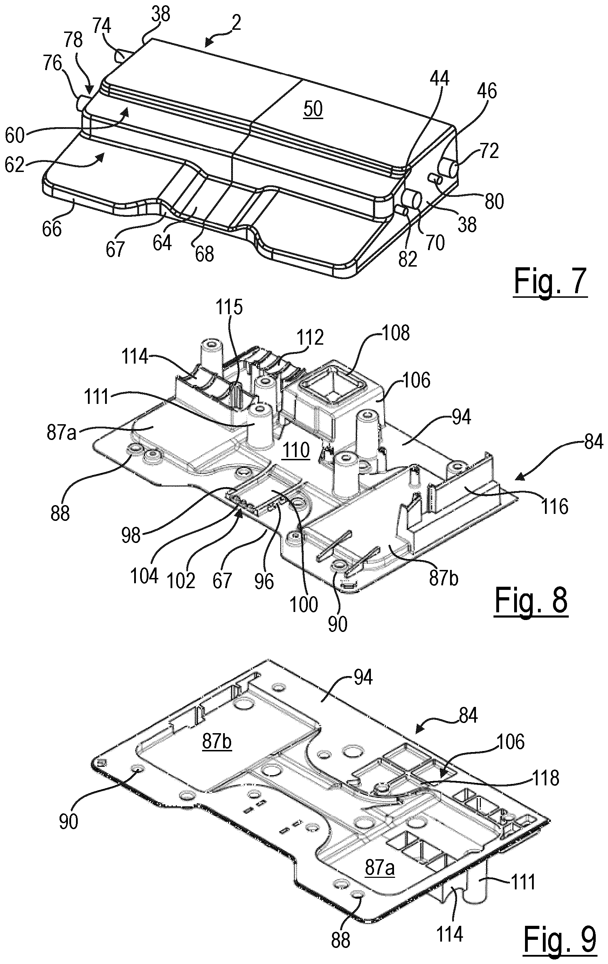

Figur 7 zeigt eine Prinzipdarstellung der vorbeschriebenen Technikbox 2, wobei die sich aus der Seitenwandung 40 durch die Durchbrüche 44, 46 hindurch erstreckenden Schwenkzapfen 70 der Schwenkgelenke 28, 30 sowie die sich aus der gegenüberliegenden Seitenwandung 39 heraus erstreckenden Schwenkgelenkkomponenten dargestellt sind. Letztere sind zum einen durch eine von einem Antriebsmotor angetriebene Gelenkwelle 74 und einen Rotationskolben 76 eines Dämpfers 78 ausgebildet.

Jeweils neben den Schwenkzapfen 70, 72 ist ein Führungspin 80 bzw. 82 angeordnet, der - wie vorstehend erläutert - die Schwenkbewegung des WC-Deckels 4 bzw. des WC-Sitzes 10 führt.

Der Schwenkzapfen 70 und der Rotationskolben 76 sind koaxial zu einander angeordnet und dienen zur Schwenklagerung des WC-Deckels 4. Dabei tauchen beide Komponenten jeweils in einen Kloben des WC-Deckels 4 ein. Auch der zugeordnete Führungspin 82 ist in einer bogenförmigen Pinführung des jeweiligen Deckel-Klobens geführt. In entsprechender Weise tauchen der koaxial zur Gelenkwelle 74 angeordnete Schwenkzapfen 72 und der Führungspin 80 in Kloben des WC-Deckels 4 ein, um diesen schwenkzulagern.

In der Darstellung gemäß Figur 7 sieht man auch die gestufte Ausgestaltung der Technikbox 2 mit der bündig zum WC-Deckel 4 ausgebildeten Deckfläche 50 und der dem gegenüber zurückgestuften Zwischenstufe 60 und Austrittsstufe 62. Im Bereich der Einschnürung 64 ist, wie vorstehend erläutert, der Luftauslass 68 ausgebildet.

Die vorbeschriebene Technikbox 2 zeichnet sich durch sehr kompaktes Design mit einem ansprechenden Äußeren und hoher Stabilität aus. Die Gründe hierfür werden im Folgenden erläutert

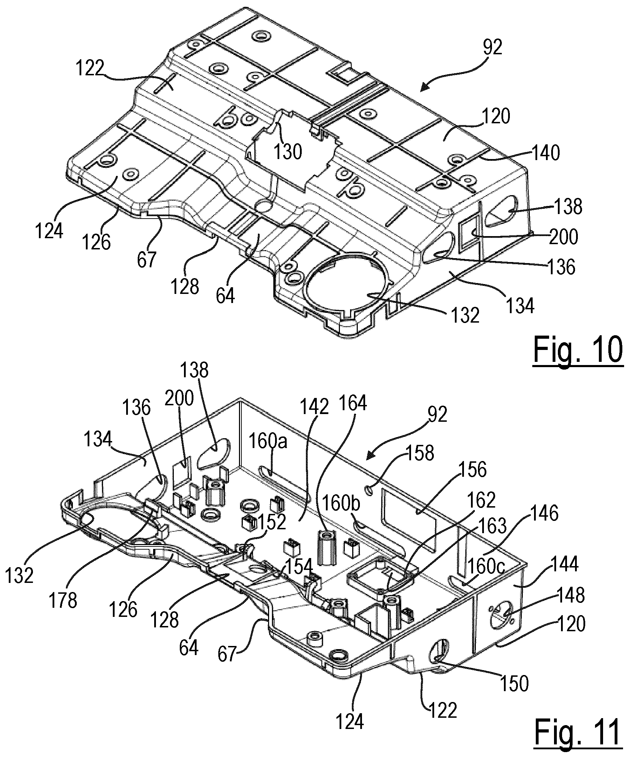

Das Gehäuse der Technikbox 2 besteht im Prinzip aus drei Bauelementen - dem Gehäuseboden 84, einem Gehäuseträger 92 (siehe Figuren 10, 11) und der vorbeschriebenen Außenschale 86 - besteht. Beim dargestellten Ausführungsbeispiel sind der Gehäuseboden 84 und der Gehäuseträger 92 mit einander verschraubt oder in sonstiger Weise mit einander verbunden, wobei diese Bauelemente so ausgebildet sind, dass sie die beim Betrieb der WC-Sitzgarnitur 1 aufnehmenden Kräfte/Momente aufnehmen können und zudem die Funktionskomponenten und die Steuerungselektronik aufnehmen/tragen. Die Außenschale 86 deckt dieses innere Gehäuse im Wesentlichen ab und bestimmt die äußere Anmutung/das Design der Technikbox 2 und hat aber praktisch keine statische Funktion.

Figur 8 zeigt eine Draufsicht auf den Gehäuseboden 84. Dieser ist entsprechend der Auflagefläche der Technikbox 2 mit einer etwa rechteckförmigen Bodenplatte 94 ausgeführt, die entlang einer Längskante (vorne in Figur 8) mit der Einwölbung 67 versehen ist. In diesem Bereich sind an der Bodenplatte 94 Wandungen 96, 98 eines Auslasskanals 100 ausgebildet, der stirnseitig durch eine Austrittswandung 102 begrenzt ist, in der vier Auslassöffnungen 104 ausgebildet sind, von denen in Figur 8 lediglich eine mit einem Bezugszeichen versehen ist. Der Auslasskanal 100 des Deodorizers mündet in einem aus der Bodenplatte 94 nach oben auskragenden Deodorizer-Gehäuse 106, in dem, wie im Folgenden noch näher erläutert, eine Kapsel/Kassette mit Duftmittel aufgenommen ist.

Das Deodorizer-Gehäuse 106 hat eine obere Flanschfläche 108, an der ein Lüfter des Deodorizers angesetzt wird. Der Auslasskanal 100 mündet im Inneren des Gehäuses 106, so dass ein von dem Lüfter geförderter Luftstrom die Kassette mit dem Duftmittel umströmt und der beladene Luftstrom dann über den Auslasskanal 100 und die Auslassöffnungen 104 in Richtung zur Keramik austritt. Der Übergangsbereich zwischen dem Auslasskanal 100 und der benachbarten Stirnwandung des Deodorizer-Gehäuses 106 ist mit einer konkaven Hohlkehle 110 ausgeführt, durch die der Querschnitt des Auslasskanals 100 zum Innenraum des Deodorizer-Gehäuses 106 hin vergrößert wird. Aus der Bodenplatte 94 kragen beim dargestellten Ausführungsbeispiel noch eine Vielzahl von Domen 111 (nur einer in Figur 8 mit einem Bezugszeichen versehen) aus, über die beispielsweise der Gehäuseträger 92 und/oder weitere Funktionskomponenten der Technikbox 2 befestigt werden können. Durch die im Gehäuseboden 84 ausgebildeten Einprägungen 87a, 87b werden Auflagen für derartige Funktionskomponenten ausgebildet. So sind beispielsweise auf der Einprägung 87a eine Lagerkonsole 112 für einen Antriebsmotor des Schwenkgelenks 28, 30 des WC-Deckels 4 und eine Dämpferkonsole 114 zur Lagerung eines Dämpfers des Schwenkgelenks 28, 30 des WC-Sitzes 10 ausgebildet. Auf der verrundeten Stützfläche der Dämpferkonsole 114 ist ein Fixierpin 115 zur Lagefixierung des Dämpfers angeordnet.

Auf der gegenüberliegenden Einprägung 87b der Bodenplatte 94 ist eine Stützwand 116 vorgesehen, an der die vorbeschriebenen Schwenkzapfen 70, 72 und die Führungspins 80, 82 seitlich abgestützt sind. Diese sind beim dargestellten Ausführungsbeispiel an dem im Folgenden beschriebenen Gehäuseträger 92 gehalten.

Von der Bodenplatte 94 kragen in das Innere des Gehäuses noch die beiden Aufnahmen 88, 90 aus, in die die beiden Gewindebolzen 16a, 16b (siehe Figur 2) eingesetzt sind. Wie im Folgenden noch näher erläutert, ist der nach oben hin offene Bereich des Auslasskanals 100 über eine Abdeckung abgedeckt, die auch einen Teil der Hohlkehle 110 überdeckt. An der Bodenplatte 94 sind noch eine Vielzahl von weiteren Stütz- und Haltestrukturen ausgebildet, die jedoch für das Verständnis der Erfindung von untergeordneten Bedeutung sind, so dass diesbezügliche Erläuterungen entbehrlich sind.

In Figur 9 ist die Unterseite des Gehäusebodens 84 dargestellt. Man erkennt, ähnlich wie in Figur 6, die beiden Einprägungen 87a, 87b, wobei sich aus der Einprägung 87a - wie vorbeschrieben - die Lagerkonsole 112 (nicht sichtbar in Figur 9) und die Dämpferkonsole 114 erstrecken, neben der einer der Dome 111 zur Befestigung des Gehäuseträgers 92 angeordnet ist.

Zwischen den beiden Einprägungen 87a, 87b erstreckt sich aus der Bodenplatte 94 nach unten (Ansicht Figur 9) das Deodorizer-Gehäuse 106, wobei im Übergangsbereich Stützrippen 118 ausgebildet sind, um das Deodorizer-Gehäuse 106 zu stabilisieren. Entsprechende Stützstrukturen sind für die weiteren Elemente des Gehäusebodens 84 in spritztechnisch optimierter Weise an der Bodenplatte 94 ausgebildet. Durch diese Stützstrukturen lässt sich der Gehäuseboden 84, insbesondere die Bodenplatte 94 mit einer hohen Steifigkeit ausbilden, so dass auch hohe Belastungen der WC-Sitzgarnitur 1 aufgefangen und in die Keramik eingeleitet werden können.

Figur 10 zeigt eine dreidimensionale Draufsicht des Gehäuseträgers 92, der beim dargestellten Ausführungsbeispiel mit dem Gehäuseboden 84 verschraubt ist und dabei im Wesentlichen auf den Domen 111 und der Bodenplatte 94 aufsitzt. Der Gehäuseträger 92 ist entsprechend der Außenkontur der Außenschale 86 ausgebildet und hat somit eine zur Keramik hin abgestufte Geometrie mit einer der Deckfläche 50 entsprechenden Großfläche 120, einer sich daran anschließenden mittleren Stufe 122 und einer zur Keramik hin abfallenden Auslassstufe 124, deren Abmessungen an die Deckfläche 50 bzw. die Zwischenstufe 60 bzw. die Austrittsstufe 62 angepasst sind, so dass die Außenschale 86 vorzugsweise flächig auf dem Gehäuseträger 92 aufliegen kann. Die Auslassstufe 124 ist an einer zur Keramik hin weisenden Stirnseite 126 wiederum mit einer rinnenförmigen Einschnürung 64 und einer Einwölbung 67 ausgebildet, in deren Scheitel der Auslasskanal 100 mündet. Dabei ist im Bereich der Einschnürung 64 eine rechteckförmige Aussparung 128 vorgesehen, die sich mit der Austrittswandung 102 und den Auslassöffnungen 104 zum Luftauslass 68 ergänzt.

Im Übergangsbereich von der den Auslasskanal 100 überdeckenden Einschnürung 64 zu dem in Figur 10 nicht sichtbaren Deodorizer-Gehäuse 106 ist im Bereich der Stufe 122 ein etwa rechteckförmiger Durchbruch ausgebildet, der als Sensoraufnahme 130 für einen Belegungssensor, beispielsweise einen Radarsensor, dient.

In dem in Figur 10 rechts von der Einschnürung 64 ausgebildeten Bereich der Auslassstufe 124 ist eine runde Ausnehmung 132 ausgebildet, in die beim dargestellten Ausführungsbeispiel eine am Gehäuseboden 84 gelagerte Senderspule für eine induktive Energieübertragung an im WC-Sitz 10 aufgenommene Komponenten eintaucht. Im Umfangsbereich der Ausnehmung 132 sind mehrere Zentrierwandungen 178 zur Lagefixierung der Senderspule ausgebildet. Diese kann gehäusebodenseitig beispielsweise auf einer Bodenfläche der Einprägung 87b (siehe Figur 8) gelagert sein.

An einer Seitenwand 134 sind längliche, sich nach links (Ansicht nach Figur 10) hin verjüngende Durchbrüche 136, 138 für die Schwenkzapfen 70, 72 und die Führungspins 80, 82 ausgebildet.

Auch der Gehäuseträger 92 ist entlang seiner Umfangsflächen mit Stützstrukturen, beispielsweise Versteifungsrippen 140, ausgeführt, über die die Steifigkeit des Gehäuseträgers 92 verbessert ist.

Figur 11 zeigt eine Innenansicht des Gehäuseträgers 92 mit Blick auf eine Trägerdeckfläche 142, die nach außen hin von der Großfläche 120 der Aussenschale 86 überdeckt ist. Diese Trägerdeckfläche 142 ist von der vorbeschriebenen Seitenwand 134, einer weiteren Seitenwand 144 und einer quer dazu verlaufenden Rückwand 146 umgeben. Die Trägerdeckfläche 142 geht dann in der vorbeschriebenen Weise in die Stufe 122 und die Auslassstufe 124 über, durch die die Höhe des Gehäuseträgers 92 stufenweise verringert ist. In der Seitenwand 144 sind Öffnungen 148, 150 für die Schwenkzapfen 70, 72 ausgebildet, die - wie im Folgenden erläutert wird - an der Seitenwand 144 gehalten sind.

Wie vorstehend erläutert, ist benachbart zur Einschnürung 64 bzw. zur Einwölbung 67 die Ausnehmung 132 für die Senderspule ausgebildet. An der Stirnseite 126 der Auslassstufe 124 ist die etwa rechteckförmige Aussparung 128 des Auslasskanals 100 vorgesehen. In der Darstellung gemäß Figur 11 erkennt man auch, dass in dem sich an die Aussparung 128 anschließenden Bereich der Auslassstufe 124 keilförmige Seitenwandungsabschnitte 152, 154 des Auslasskanals 100 ausgebildet sind.

In der Rückwandung 146 ist ein Fenster 156 für die Schublade 24 des Deodorizer und seitlich neben diesem Fenster 156 ist eine Kabeldurchführung 158 für die Leitung 22 ausgebildet. In dem an die Trägerdeckfläche 142 angrenzenden Bereich der Rückwandung 146 sind drei Lichtaustrittsdurchbrüche 160a, 160b, 160c ausgebildet, die mit dem Lichtaustrittsöffnungen 36a, 36b, 36c der Außenschale 86 fluchten und durch die hindurch das vorbeschriebene Leuchtmittel Licht abgibt.

In der Ansicht gemäß Figur 11 unterhalb des Fensters 156 ist auf der Trägerdeckfläche 142 eine Halterung 162 für den Lüfter des Deodorizers vorgesehen. Diese Halterung 162 ist beim dargestellten Ausführungsbeispiel rahmenförmig ausgebildet, wobei über eine Ansaugöffnung 163 eine Verbindung zum Lufteinlass 32 hergestellt ist. Der Lüfter kann mit den Eckbereichen der Halterung 162 verschraubt werden.

Mit dem Bezugszeichen 164 ist beispielhaft ein aus der Trägerdeckfläche 142 auskragender Stützdom bezeichnet, über den weitere Funktionskomponenten der Technikbox 2 gehalten werden können oder über den eine Verbindung mit dem Gehäuseboden 84, beispielsweise durch Schrauben, erfolgt.

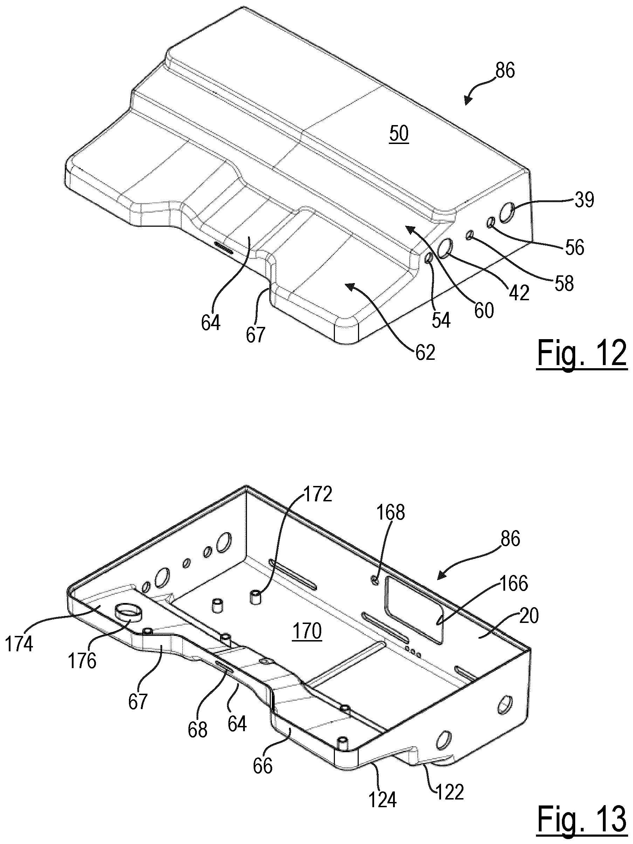

Figur 12 zeigt eine Draufsicht auf die Außenschale 86. Diese Ansicht entspricht im Wesentlichen der Gesamtansicht der Technikbox 2 gemäß Figur 4, so dass weitere Erläuterungen entbehrlich sind und im Hinblick auf die einzelnen Elemente auf diese Figur 4 verwiesen werden kann. Dementsprechend ist die Außenschale 86 nach außen hin glattflächig und im Hinblick auf ein optimales Design ausgelegt. Entsprechend ist auch das in Figur 13 dargestellte Innere der Außenschale 86 mit relativ wenig Strukturelementen ausgeführt, so dass beim Herstellen, vorzugsweise beim Spritzgießen aufgrund nicht vorhandener Materialanhäufungen eine optimale Qualität der Sichtflächen gewährleistet ist. In der Rückwandung 20 ist eine Schubladenöffnung 166 ausgebildet, die mit dem Fenster 156 des Gehäuseträgers 92 fluchtet und in die die im Folgenden noch näher erläuterte Schublade 24 eingesetzt wird. Neben der Schubladenöffnung 166 ist eine mit der Kabeldurchführung 158 fluchtende Durchführung 168 für die Leitung 22 ausgebildet. Auf einer Außenschalendeckfläche 170 und den Großflächen der Stufe 122 und der Auslassstufe 124 sind einige wenige Hohlstutzen 172 ausgebildet, über die die Außenschale 86 auf dem Gehäuseträger 92 abgestützt ist. Der Luftauslass 68 mündet in der vorbeschriebenen Weise in der Stirnfläche 66 der Einschnürung 64 bzw. der Einwölbung 67. An einer Innenfläche 174 der Auslassstufe 124 ist ein Fixierring 176 für die Senderspule angeordnet ist. Dieser Fixierring 176 ist koaxial zur Ausnehmung 132 ausgebildet.

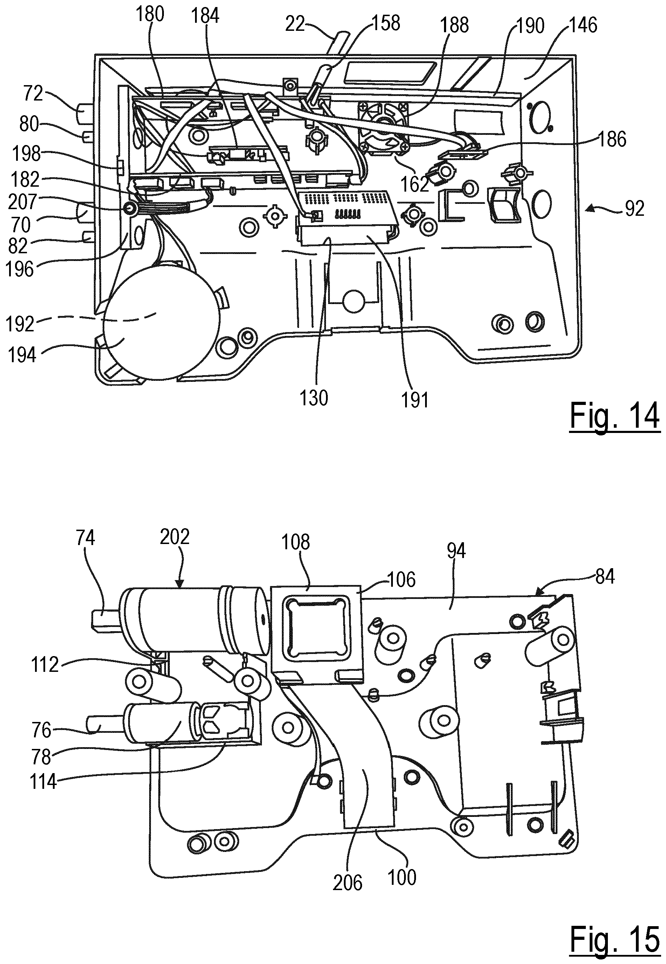

Anhand der Figuren 8 bis 13 wird vorstehend der Grundaufbau des dreiteiligen Gehäuses der Technikbox 2 erläutert. In den folgenden Figuren 14 und 15 sind einige der beschriebenen Funktionskomponenten und Teile der Steuerungselektronik in die tragenden Strukturelemente, d.h. in den Gehäuseträger 92 (Figur 14) und den Gehäuseboden 84 (Figur 15) eingebaut.

In Figur 14 ist der Gehäuseträger 92 in einer etwa Figur 11 entsprechenden Ansicht dargestellt. Man sieht die durch die Kabeldurchführung 158 in das Gehäuseinnere geführte Leitung 22, über die Steuerplatinen 180, 182, 184 und 186 mit elektrischer Energie versorgt werden. Über die letztgenannte Steuerplatine 186 wird ein Lüfter 188 angesteuert, der - wie vorstehend erläutert - auf der Halterung 162 des Gehäuseträgers 92 befestigt ist. Über die Leistungselektronik mit den Steuerplatinen 180, 182, 184, 186 erfolgt auch die Ansteuerung eines Lichtmoduls 190, das im Bereich der Rückwandung 146 befestigt ist und das Licht über die vorbeschriebenen Lichtaustrittsöffnungen 36 der Außenschale 86 abgibt.

Die Steuerungselektronik ist des Weiteren im Hinblick auf die Energie- und Signalübertragung an einige Sensoren, beispielsweise an den vorbeschriebenen Radarsensor 191, angeschlossen, der in die Sensoraufnahme 130 eingesetzt ist. Über diesen Radarsensor 191 kann die Annäherung eines Nutzers/einer Nutzerin an die WC-Sitzgarnitur 1 erfasst werden, so dass nach der Erfassung die Funktionskomponenten in geeigneter Weise, beispielsweise zum Anheben des WC-Deckels 4 oder dergleichen, angesteuert werden können.

In Figur 14 ist auch eine Senderspule 192 gezeigt, die allerdings von einer Abdeckung 194 überdeckt ist. Diese Senderspule 192 dient zur induktiven Signal- /Energieübertragung an in dem WC-Sitz 10 oder in dem WC-Deckel 4 integrierte Komponenten, bspw. eine Sitzheizung.

Beim dargestellten Ausführungsbeispiel sind die Schwenkzapfen 70, 72 und die benachbart dazu ausgebildeten Führungspins 80, 82 an einer Platte 196 gehalten, die an der Stützwand 116 des Gehäusebodens 84 und an der Innenseite der Seitenwand 134 und der Trägerdeckfläche 142 des Gehäuseträgers 92 abgestützt ist. In diesem Bereich kann auch ein weiterer Sensor 198 vorgesehen sein, über den beispielsweise die Schwenkposition des WC-Deckels 4 und/oder des WC-Sitzes 10 erfasst wird und der in ein Sensorfenster 200/(siehe insbesondere Figur 11) eingesetzt ist. In Abhängigkeit von dessen Signal kann beispielsweise der in Figur 15 dargestellte Antriebsmotor 202 der Gelenkwelle 74 angesteuert werden.

Bei dem dargestellten Ausführungsbeispiel ist optional ein Belegsensor 206 vorgesehen, über den die Nutzung des WC-Sitzes 10 erfasst wird. Über diesen Belegsensor 207 wird die Belastung des sitzseitigen Schwenkzapfens 70, 72 erfasst und über die Steuerelektronik/Leitungselektronik zur Steuerung von Funktionskomponenten, beispielsweise einer Sitzheizung oder dergleichen, verwendet.

In der Figur 15 ist der Gehäuseboden 84 in einer Figur 8 entsprechenden Ansicht dargestellt. Demgemäß ist der Antriebsmotor 202 auf die Lagerkonsole 112 der Bodenplatte 94 aufgesetzt und kragt mit der Gelenkwelle 74 aus der Technikbox 2 aus. Diese Gelenkwelle 74 ist drehfest mit einem Kloben des WC-Deckels 4 verbunden, so dass bei Ansteuerung des Antriebsmotors 202 der WC-Deckel 4 geöffnet (angehoben) oder geschlossen (abgesenkt) wird. Der Motor bzw. dessen Getriebe ist vorzugsweise so ausgelegt, dass die Selbsthemmung relativ gering ist, so dass die WC-Sitzgarnitur 1 auch stromlos von Hand geöffnet oder geschlossen werden kann. Dieser Antriebsmotor 202 kann im Prinzip auch zum Öffnen und Schließen des WC-Sitzes 10 verwendet werden. Beim konkreten Ausführungsbeispiel ist der Antriebsmotor 202 lediglich dem WC-Deckel 4 zugeordnet.

Die Absenkbewegung des WC-Sitzes 10 von einer Öffnungsstellung in Richtung einer Schließstellung (auf der Keramik aufliegend) wird durch einen herkömmlichen WC-Sitzgelenk-Dämpfer 204 durchgeführt, dessen Rotationskolben 76 parallel zur Gelenkwelle 74 aus der Technikbox 2 auskragt. Die beiden Schwenkzapfen 70, 72 sind koaxial zur Gelenkwelle 74 bzw. zum Rotationskolben 76 angeordnet. Der Dämpfer 204 ist auf der Dämpferkonsole 114 der Bodenplatte 94 abgestützt. In der Darstellung gemäß Figur 15 ist auch eine Abdeckung 206 sichtbar, die den auf der Bodenplatte 94 ausgebildeten Auslasskanal 100 und die sich daran anschließende Hohlkehle 110 überdeckt und die an dem Deodorizer-Gehäuse 106 anliegt. In dieser Darstellung sieht man auch dessen Flanschfläche 108, auf der der Lüfter 188 dichtend aufliegt. Weitere Einzelheiten der Luftführung werden im Folgenden noch näher erläutert.

In die Anordnung gemäß den Figuren 14 und 15 kann des Weiteren noch ein Funkmodul integriert sein, so dass die einzelnen Funktionskomponenten über ein externes Steuerungsgerät, beispielsweise ein Hand-Held oder ein Smartphone ansteuerbar sind. Über dieses Funkmodul können im Prinzip auch weitere Daten, beispielsweise Wetterdaten oder dergleichen, geladen werden, die dann vom Nutzer/der Nutzerin abgerufen werden können. Auch eine Signalverarbeitung/-auswertung der von Sensoren erfassten Vitalparameter oder sonstiger Signale kann über dieses externe Steuergerät erfolgen.

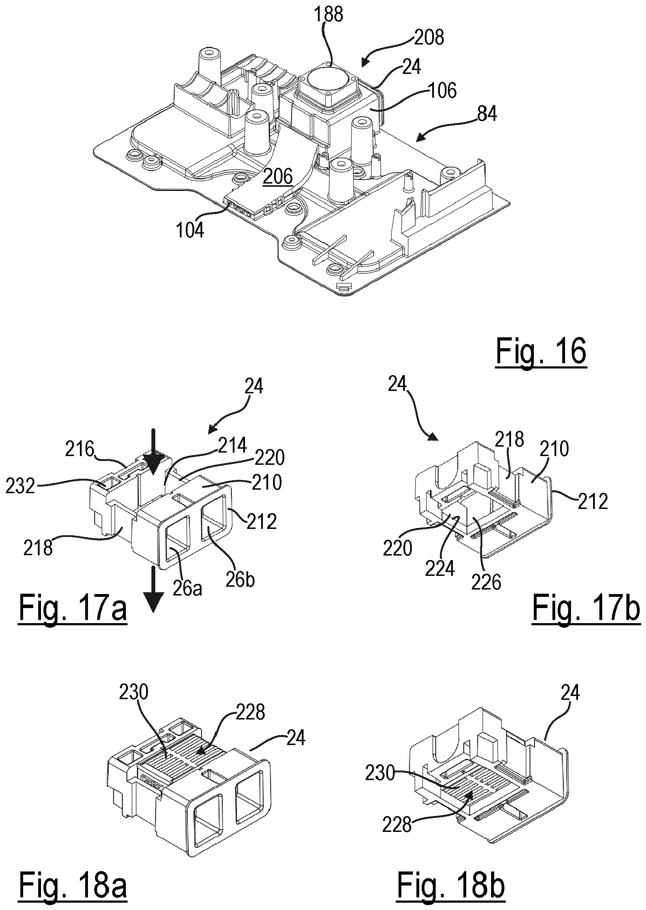

Wie vorstehend ausgeführt, ist die WC-Sitzgarnitur 1, insbesondere die Technikbox 2 mit einem Deodorizer ausgeführt sein. Dieser ist in der Figur 16 mit dem Bezugszeichen 208 versehen. Bei diesem Ausführungsbeispiel ist der Deodorizer 208 im Wesentlichen auf dem Gehäuseboden 84 angeordnet. Prinzipiell kann dieser jedoch auch in anderer Form in die Technikbox 2 integriert sein. Figur 16 zeigt eine Figur 8 entsprechende Darstellung des Gehäusebodens 84, wobei im Unterschied zu dieser Darstellung in das Deodorizer-Gehäuse 106 die Schublade 24 eingesetzt ist. Des Weiteren ist der Auslasskanal 100 mit den Auslassöffnungen 104 von der konkav gebogenen Abdeckung 206 überdeckt. In der Darstellung gemäß Figur 16 ist des Weiteren der Lüfter 188 auf das Deodorizer-Gehäuse 106 aufgesetzt.

Wie erläutert, dient die Schublade 24 dazu, ein Beduftungsmittel aufzunehmen. Figuren 17a, 17b zeigen eine Draufsicht bzw. eine Unteransicht der Schublade 24, die in eine Führung des Deodorizer-Gehäuses 106 eingeschoben wird. Wie erläutert, hat die Schublade 24 zwei Griffmulden 26a, 26b, die an einem Griffkörper 210 ausgebildet sind. Die zum Nutzer/der Nutzerin weisende Stirnfläche des Griffkörpers 210 ist durch eine Stirnwandung 212 gebildet, die im eingesetzten Zustand der Schublade 24 bündig mit der Rückwandung 20 der Technikbox 2 abschließt. Diese Stirnwandung 212 steht seitlich etwas über den Außenumfang des Griffkörpers 210 hinaus, so dass die Führung der Schublade 24 in der Technikbox 2, genauer gesagt in der Außenschale 86, dem Gehäuseträger 92 und dem Gehäuseboden 84 abgedeckt ist. Im Anschluss an den Griffkörper 210 ist ein Aufnahmeraum 214 für das Beduftungsmittel ausgebildet, der rückseitig durch eine Querwandung 216 begrenzt ist. Die seitliche Begrenzung erfolgt über Seitenwandungen 218, 220, die sich zwischen dem Griffkörper 210 und der Querwandung 216 erstrecken.

Wie der Unteransicht gemäß Figur 17b entnehmbar, ist der Aufnahmeraum 214 nach unten hin durch einen Bodenrahmen 224 begrenzt, wobei eine von diesem umgriffene Austrittsöffnung 226 eine Durchströmung der Schublade 24 in Vertikalrichtung (siehe Pfeil in Figur 17a). Beim dargestellten Ausführungsbeispiel ist das Beduftungsmittel in Kassetten/Kapseln 228 aufgenommen, die gemäß den Figuren 18a, 18b passgenau in den Aufnahmeraum 214 der Schublade 24 eingesetzt werden können. Die Kassetten 228 sind dabei mit einem mit dem Beduftungsmittel beladenen Granulat gefüllt, so dass die Luft beim Durchströmen der Kassette 228 mit dem Duftstoff beladen wird. Beim dargestellten Ausführungsbeispiel wird die Durchströmung der Kassette 228 durch am Außenumfang ausgebildete Schlitze 230 ermöglicht, so dass die Luft ohne größeren Druckverlust die Schublade 24 in Pfeilrichtung durchströmen kann. Derartige Kassetten/Kapseln 228 sind beispielsweise bei Belüftungssystemen für Fahrzeuge/Räume bekannt, so dass weitere Ausführungen entbehrlich sind.

Je nach Art der verwendeten Kassetten/Kapseln 228 können Schubladen 24 mit unterschiedlich ausgeformten Aufnahmeräumen 214 bereitgestellt werden. Prinzipiell ist es jedoch auch möglich, den Aufnahmeraum 214 durch eingelegte Adapter an unterschiedliche Kassetten/Kapseln 228 anzupassen. Beim dargestellten Ausführungsbeispiel sind die Querwandung 216 und der Griffkörper 210 jeweils als Hohlkörper ausgebildet, wobei zur Vermeidung von Materialanhäufungen Hohlkammerstrukturen eingebracht sind - diese sind in Figur 17a beispielhaft mit dem Bezugszeichen 232 versehen.

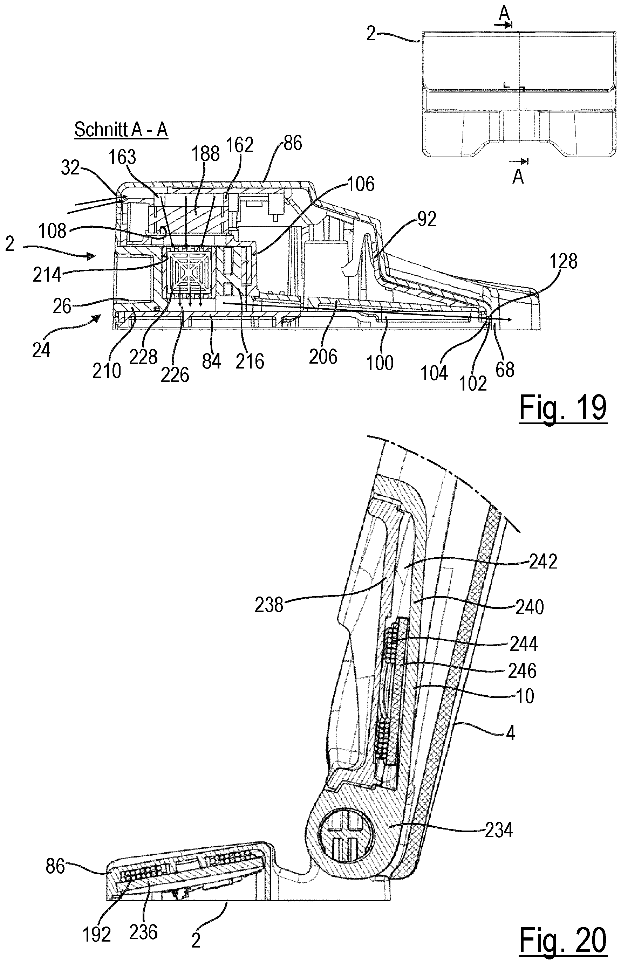

Figur 19 zeigt einen Schnitt A-A durch die Technikbox 2, wobei der Schnittverlauf in Figur 19 oben rechts eingeblendet ist. D.h. die Schnittebene hat zwei in Querrichtung (waagerecht in Figur 19) versetzte Schnittbereiche.

Dementsprechend sieht man in dem Schnitt A-A den gemäß den vorstehenden Ausführungen ausgebildeten Gehäuseboden 84, der mit dem Gehäuseträger 92 verschraubt oder in sonstiger Weise verbunden ist. Nach außen hin ist das vom Gehäuseboden 84 und vom Gehäuseträger 92 gebildete Innengehäuse von der Außenschale 86 überdeckt. Wie eingangs erläutert, ist in dieser Außenschale 86 der Lufteinlass 32 ausgebildet, durch den Luft in das Innere der Technikbox 2 eintreten kann. Gemäß den vorstehenden Ausführungen ist in das Deodorizer-Gehäuse 106 die Schublade 24 mit der Kassette 228 eingesetzt. In dem Schnitt gemäß Figur 19 sieht man den mit einer der Griffmulden 26 ausgebildeten Griffkörper 210 und die Querwandung 216, zwischen denen der Aufnahmeraum 214 für die Kassette 228 ausgebildet ist. Wie erläutert, öffnet sich die Schublade 24 in der Darstellung gemäß Figur 19 nach unten hin über die Austrittsöffnung 226 zum Innenraum des Deodorizer-Gehäuses 106, das in Strömungsverbindung mit dem Auslasskanal 100 steht, der nach oben hin durch die Abdeckung 206 abgedeckt ist. Der Auslasskanal 100 mündet in dem Luftauslass 68, der einerseits durch die Aussparung 128 des Gehäuseträgers 92 und andererseits durch die Austrittswandung 102 mit den Auslassöffnungen 104 begrenzt ist. Der Lüfter 188 ist, wie vorstehend erläutert, am Gehäuseträger 92 befestigt und liegt auf der Flanschfläche 108 des Deodorizer-Gehäuses 106 auf.

Bei Ansteuerung des Lüfters 188 über die beschriebene Steuer- /Leistungselektronik wird Luft, wie in Figur 19 dargestellt, durch den Lufteinlass 32 in das Innere der Technikbox 2 angesaugt und tritt durch die Ansaugöffnung 163 in das Innere des Deodorizer-Gehäuses 106 ein. Der Luftstrom wird dann über den Lüfter 188 und die Umfangswandungen des Deodorizer-Gehäuses 106 durch die in der Schublade 24 aufgenommene Kassette 228 geführt, so dass die Luft mit dem Beduftungsmittel/Duftstoff beladen wird. Diese beladene Luft tritt dann über die Austrittsöffnung 226 der Schublade 24 in den unteren Teil des Deodorizer-Gehäuses 106 ein und strömt von dort in Richtung des Auslasskanals 100, bis sie schließlich über den Luftauslass 68 aus der Technikbox 2 in Richtung zur Keramik strömt.

Wie vorstehend erläutert, kann die Betätigung des Lüfters 188 in Abhängigkeit von dem Signal eines Belegsensors oder dergleichen erfolgen. Prinzipiell ist es natürlich auch möglich, den Lüfter 188 über ein Steuergerät nach Art einer Fernbedienung anzusteuern. Bei einfacheren Lösungen kann auch eine manuelle Betätigung des Lüfters 188 mit Hilfe eines Tasters oder Schalters erfolgen.

Eine Besonderheit der vorstehend beschriebenen Luftstromführung besteht darin, dass diese im Wesentlichen entlang einer Vertikalebene (Schnittebene in Figur 19) erfolgt, wobei die Luftzufuhr in Vertikalrichtung gesehen oberhalb des Luftaustritts erfolgt, wobei die Kassette 228 in Vertikalrichtung durchströmt wird. Prinzipiell ist je nach Ausgestaltung der Technikbox 2 auch eine andere Strömungsführung möglich. Die vorbeschriebene Strömungsführung hat jedoch den Vorteil, dass der Deodorizer 208 sehr kompakt baut, so dass seitlich davon genügend Platz für weitere Komponenten der WC-Sitzgarnitur 1 verbleibt.

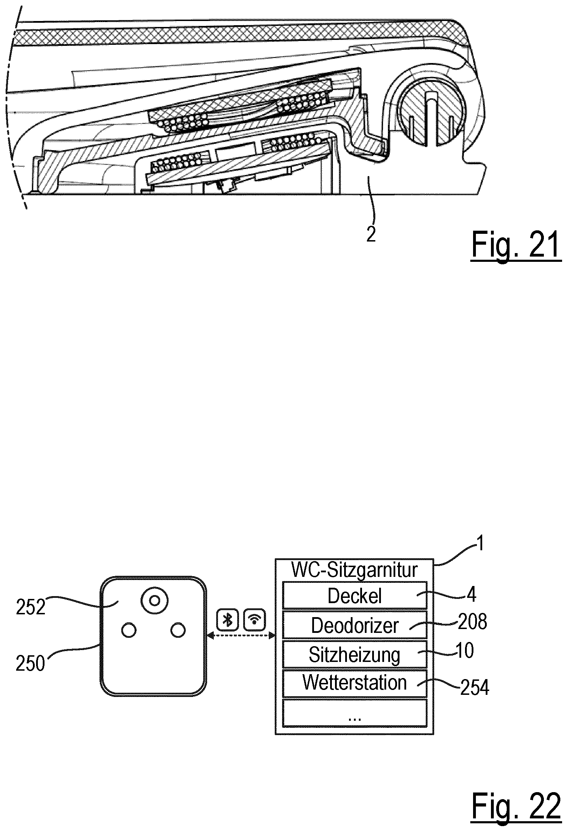

Das Grundkonzept der induktiven Energie-/Datenübertragung wird im Folgenden anhand der Figuren 20, 21 erläutert, wobei ergänzend auf den eingangs beschriebenen Stand der Technik gemäß den Dokumenten DE 10 2019 110 281 A1 und DE 10 2019 110 287 A1 verwiesen werden kann.

Figur 20 zeigt ein Ausführungsbeispiel einer erfindungsgemäßen WC-Sitzgarnitur 1, wobei allerdings die Technikbox 2 mit geringeren Abmessungen als bei den vorbeschriebenen Ausführungsbeispielen ausgeführt ist. Wie erläutert, sind an die Technikbox 2 ein WC-Deckel 4 und ein WC-Sitz 10 angelenkt, wobei die eingangs beschriebenen Schwenkgelenke (Gelenkwelle 74, Rotationskolben 76 des Dämpfers 78, Schwenkzapfen 70, 72) jeweils in Kloben 234 des WC-Sitzes 10 und des WC-Deckels 4 eingreifen, um diese schwenkzulagern. In der Figur 20 ist lediglich der Kloben 234 des WC-Sitzes 10 mit einem Bezugszeichen versehen.

Wie vorstehend erläutert, ist in der Technikbox 2 die Senderspule 192 gehalten, die beispielsweise auf einem Träger 236 angeordnet und in der Technikbox 2 lagefixiert ist. Dies erfolgt vorzugsweise nahe in dem Bereich der Außenschale 86, der zum WC-Sitz 10 in seiner Schießstellung zugewandt ist. Die Energieversorgung und Leistungselektronik für die Senderspule 192 sind, wie erläutert, in der Technikbox 2 aufgenommen.

Beim dargestellten Ausführungsbeispiel ist der WC-Sitz 10 mit zwei Schalen 238, 240 ausgeführt, die gemeinsam einen Aufnahmeraum 242 für eine Empfängerspule 244 bilden. Diese ist beim dargestellten Ausführungsbeispiel ebenfalls auf einem Träger 246 angeordnet und im Aufnahmeraum 242 lagefixiert. Prinzipiell kann der WC-Sitz 10 jedoch auch einteilig ausgebildet sein, wobei dann die Empfängerspule 244 beim Pressen direkt mitverpresst wird.

Bei der in Figur 20 dargestellten Relativposition von Empfängerspule 244 und Senderspule 192 findet keine induktive Energieübertragung statt. Bei abgesenktem WC-Sitz 10 ist die Empfängerspule 244 gemäß Figur 21 so mit Bezug zur Senderspule 192 ausgerichtet, dass über die beiden Spulen ein induktives elektromagnetisches Wechselfeld erzeugt wird. Die Energieübertragung erfolgt dann durch Gegeninduktion zwischen den beiden Spulen (Empfängerspule 244 und Senderspule 192), wobei die in der Empfängerspule 244 induzierte Wechselspannung über die Steuerelektronik gleichgerichtet und an die im WC-Sitz 10 integrierten Funktionskomponenten, beispielsweise eine Sitzheizung, Sensoren, LED's oder dergleichen, weitergegeben werden kann. Diese induktive Energieübertragung findet, wie oben dargelegt, nur bei abgesenktem WC-Sitz 10 statt. Sobald dieser nach oben in die Öffnungsstellung geschwenkt wird, ist die induktive Kopplung zwischen den Spulen 244, 192 aufgehoben, so dass keine Energieübertragung und somit auch keine Versorgung der Verbraucher erfolgt. Selbstverständlich kann im WC-Sitz 10 auch ein Energiespeicher, beispielsweise ein Kondensator oder ein Akkumulator, aufgenommen werden, um die Funktionskomponenten auch bei angehobenem WC-Sitz 10 über einen vorbestimmten Zeitraum mit Energie zu versorgen.

Die aus dem Stand der Technik bekannten WC-Sitzgarnituren werden entweder über die vorbeschriebenen Sensoren oder aber durch Betätigung von Tastern/Schaltern gesteuert. Bekannt sind auch Lösungen, mit Tasten- oder Folienfernbedienungen, bei denen die Steuersignale drahtlos an die WC-Sitzgarnitur übertragen werden.

Bei einem Ausführungsbeispiel einer erfindungsgemäßen WC-Sitzgarnitur wird ein mit einem Display ausgeführtes Bedienelement, beispielsweise ein Tablet oder ein Smartphone, verwendet, über das die vorstehend erläuterten Funktionskomponenten angesteuert werden können, wobei über den Bildschirm auch eine Überwachung der Funktion und eine Auswertung von über die Sensorik erfassten Daten ermöglicht ist. Das Grundkonzept eines derartigen Systems ist in Figur 22 dargestellt. Demgemäß ist das Bedienelement beispielsweise als Tablet oder Handheld 250 mit einem Touch-Display 252 ausgeführt, über das die Funktionen der WC-Sitzgarnitur 1 gesteuert werden können. Wie vorstehend erläutert, ist in der WC-Sitzgarnitur 1 ein Funkmodul integriert, so dass ein Datenaustausch zwischen der WC-Sitzgarnitur 1 und dem Handheld 250 möglich ist. Dieser Datenaustausch kann beispielsweise über Bluetooth oder WLAN oder in sonstiger Weise erfolgen. Durch entsprechende Betätigung des Handheld 250 können dann beispielsweise der WC-Deckel 4, der Deodorizer 208, eine Sitzheizung oder sonstige Komponenten des WC-Deckels 4 angesteuert werden oder es können Daten von einer anderen Station, beispielsweise von einer Wetterstation 254 abgerufen werden, um diese während der Nutzung auf Display 252 anzuzeigen.

Selbstverständlich können anstelle derartiger Wetterdaten auch sonstige Nachrichten oder Informationen abgerufen und auf dem Display 252 angezeigt werden.

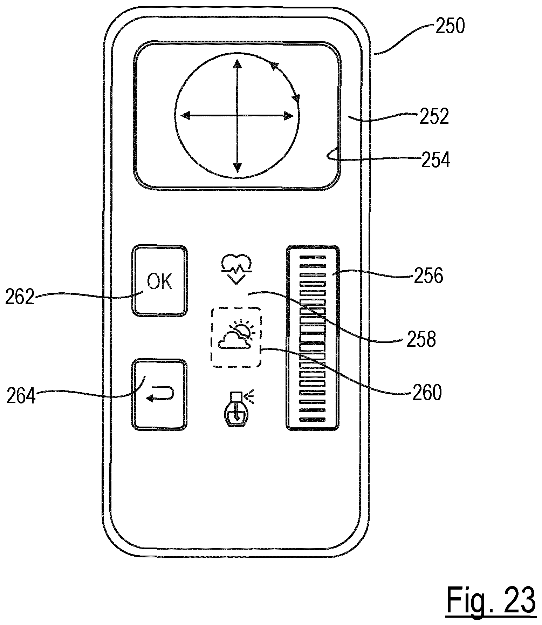

Figur 23 zeigt eine mögliche Ausgestaltung eines derartigen Handheld 250. Demgemäß hat dieses eine, vorzugsweise touch-sensitive, Bedienfläche 252, auf der beispielsweise ein Gestenfeld 254 ausgebildet ist. Dies ermöglicht es, unterschiedliche Single-Touch-Gesten mit Funktionen zu verknüpfen, so dass über entsprechende Bewegung mit einem oder mehreren Fingern auf der Gestenfläche 254 die betreffende Funktion zur Steuerung der WC-Sitzgarnitur 1 abgerufen werden kann. Derartige Single-Touch-Gesten können beispielsweise Bewegungen nach oben, unten, links, rechts oder entlang eines Kreises im Uhrzeigersinn oder gegen den Uhrzeigersinn sein, so dass eine Vielzahl von eindeutigen Gesten zur Steuerung der Funktionen zur Verfügung steht.

Auf der Bedienfläche 252 des beschriebenen Ausführungsbeispiels sind weitere touch-sensitive Bedienelemente realisiert. Mit dem Feld 256 ist eine Scrollfläche oder ein Schieber gekennzeichnet, über den durch eine im Handheld 250 abgelegte Menüstruktur navigiert werden kann. Diese Menüstruktur kann auf einem mittigen Display 258 angezeigt werden, wobei durch Betätigung der Scrollfläche 258 die jeweilige Funktion in den Auswahlbereich 260 gebracht wird. Dabei kann die Menüstruktur zur Anzeige von verschiedenen Daten oder zur Auswahl von verschiedenen Funktionen ausgewählt werden. Es wird beispielsweise in der in Figur 23 gewählten Einstellung die Verbindung zur Wetterstation hergestellt, so dass entsprechende Wetterdaten abgerufen werden. Bei Auswahl des in Figur 23 darüberliegenden Symbols werden Vitalparameter, wie beispielsweise der Blutdruck die Herzfrequenz oder dergleichen abgerufen und verarbeitet. Mit der in Figur 23 unten liegenden Auswahl wird der Deodorizer 208 angesteuert. In entsprechender Weise können Symbole zur Betätigung der Sitzheizung, zum Anheben und Absenken des WC-Deckels 4 und/oder des WC-Sitzes 10 oder zur Steuerung der Beleuchtung ausgewählt werden. Die endgültige Auswahl erfolgt dabei über einen OK-Button 262. In Figur 23 ist unterhalb des OK-Buttons 262 noch ein Zurück-(Return)-Button 264 dargestellt. Über ein derartiges Handheld 250 lassen sich somit bei entsprechender Auslegung der Software alle wesentlichen Funktionen einer WC-Sitzgarnitur 1 steuern und ggf. auch eine Auswertung von gemessenen Daten durchführen.

Dabei wird es bevorzugt, wenn das Handheld 250 mit einem fest integrierten Akku versehen ist, der beispielsweise über eine Mikro-USB-Schnittstelle oder dgl. der WC-Sitzgarnitur 1 oder aber auch induktiv aufgeladen werden kann.

Über dieses Handheld 250 kann auch eine Bedienungsanweisung der WC-Sitzgarnitur 1 oder aber auch Wartungsanweisungen seitens des Herstellers abgerufen werden.

Wie erläutert, kann die Funktion des Handheld 250 auch durch eine auf ein Smartphone ladbare App übernommen werden, durch die dann entsprechende Signale und Eingaben des Nutzers/der Nutzerin zur Steuerung/Auswertung an die WC-Sitzgarnitur 1 versendet werden.

Offenbart ist eine WC-Sitzgarnitur mit einer Technikbox, in der Funktionskomponenten und eine Steuerelektronik der WC-Sitzgarnitur aufgenommen sind. Die Ansteuerung der WC-Sitzgarnitur erfolgt vorzugsweise über ein externes Bediengerät.

### Bezugszeichenliste:

- 1: WC-Sitzgarnitur
- 2: Technikbox
- 4: WC-Deckel
- 6: Puffer
- 8: Puffer
- 10: WC-Sitz
- 12: Befestigungsmittel
- 14: Befestigungsmittel
- 16: Gewindebolzen
- 18: Mutter
- 20: Rückwandung
- 22: Leitung
- 24: Schublade
- 26: Griffmulde
- 28: Schwenkgelenk
- 30: Schwenkgelenk
- 32: Lufteinlass
- 34: Einlassausnehmung
- 36: Lichtaustrittsöffnung
- 37: Glaseinsatz
- 38: Seitenwandung
- 39: Durchbruch
- 40: Seitenwandung
- 42: Durchbruch
- 44: Durchbruch
- 46: Durchbruch
- 48: Durchführung
- 50: Deckfläche
- 52: Großfläche
- 54: Ausnehmung
- 56: Ausnehmung
- 58: Durchführung
- 60: Zwischenstufe
- 62: Austrittsstufe
- 64: Einschnürung
- 66: Stirnfläche
- 67: Einwölbung
- 68: Luftauslass
- 70: Schwenkzapfen
- 72: Schwenkzapfen
- 74: Gelenkwelle
- 76: Rotationskolben
- 78: Dämpfer
- 80: Führungspin
- 82: Führungspin
- 84: Gehäuseboden
- 86: Außenschale
- 87: Einprägung
- 88: Aufnahme
- 90: Aufnahme
- 92: Gehäuseträger
- 94: Bodenplatte
- 96: Wandung
- 98: Wandung
- 100: Auslasskanal
- 102: Austrittswandung
- 104: Auslassöffnungen
- 106: Deodorizer-Gehäuse
- 108: Flanschfläche
- 110: Hohlkehle
- 111: Dom
- 112: Lagerkonsole
- 114: Dämpferkonsole
- 115: Fixierpin
- 116: Stützwand
- 118: Stützrippe
- 120: Großfläche
- 122: Stufe
- 124: Auslassstufe
- 126: Stirnseite
- 128: Aussparung
- 130: Sensoraufnahme
- 132: Ausnehmung
- 134: Seitenwand
- 136: Durchbruch
- 138: Durchbruch
- 140: Versteifungsrippen
- 142: Trägerdeckfläche
- 144: Seitenwand
- 146: Rückwand
- 148: Öffnung
- 150: Öffnung
- 152: Seitenwandungsabschnitt
- 154: Seitenwandungsabschnitt
- 156: Fenster
- 158: Kabeldurchführung
- 160: Lichtaustrittsdurchbruch
- 162: Halterung
- 163: Ansaugöffnung
- 164: Stützdom
- 166: Schubladenöffnung
- 168: Durchführung
- 170: Außenschalendeckfläche
- 172: Hohlstutzen
- 174: Innenfläche
- 176: Fixierring
- 178: Zentrierwand
- 180: Steuerplatine
- 182: Steuerplatine
- 184: Steuerplatine
- 186: Steuerplatine
- 188: Lüfter
- 190: Lichtmodul
- 191: Radarsensor
- 192: Senderspule
- 194: Abdeckung
- 196: Platte
- 198: Sensor
- 200: Sensorfenster
- 202: Antriebsmotor
- 206: Abdeckung
- 207: Belegsensor
- 208: Deodorizer
- 210: Griffkörper
- 212: Stirnwandung
- 214: Aufnahmeraum
- 216: Querwandung
- 218: Seitenwandung
- 220: Seitenwandung
- 224: Bodenrahmen
- 226: Austrittsöffnung
- 228: Kassette/Kapsel
- 230: Schlitz
- 232: Hohlkammerstruktur
- 234: Kloben
- 236: Träger
- 238: Schale
- 240: Schale
- 242: Aufnahmeraum
- 244: Empfängerspule
- 246: Träger
- 250: Handheld
- 252: Bedienfläche (Display)
- 254: Gestenfeld
- 256: Scrollfläche
- 258: Display
- 260: Auswahlbereich
- 262: OK-Button
- 264: Zurück-(Return)-Button

## Patentansprüche

1. Technikbox für eine WC-Sitzgarnitur (1), die über Schwenkgelenke (28, 30) mit der Technikbox (2) verbunden ist, die ihrerseits über Befestigungsmittel (12, 14) an einer Keramik festlegbar ist, wobei in einem Gehäuse der Technikbox (2) Funktionskomponenten und eine Steuerungselektronik der WC-Sitzgarnitur (1) angeordnet sind, wobei ein Kommunikationsmodul in der Technikbox (2) aufgenommen ist, dem ein externes Bedienelement, beispielsweise ein Handheld (250), zugeordnet ist, das ausgelegt ist, auf der Basis einer kontaktlosen Kommunikation mit dem in die Technikbox integrierten Kommunikationsmodul über das Kommunikationsmodul die Funktionskomponenten und/oder die Steuerungselektronik der WC-Sitzgarnitur (1) anzusteuern und/oder Informationen anzuzeigen und/oder Daten auszuwerten.

2. Technikbox nach Patentanspruch 1, wobei die kontaktlose Signal-/Datenübertragung beispielsweise mittels WLAN, Wifi, Bluetooth, ZigBee, NFC, Wibree, BLE, IrDA, WIS oder Funk erfolgt.

3. Technikbox nach Patentanspruch 1 oder 2, wobei das Bedienelement eine Bedienfläche (252) mit mehreren anwählbaren Funktionsfeldern hat.

4. Technikbox nach Patentanspruch 3, wobei die Bedienfläche (252) touch-sensitiv ist.

5. Technikbox nach Patentanspruch 4, wobei die Bedienfläche (252) ein Gestenfeld (254), ein Scrollfeld (256), ein Display (258), einen OK-Button (262) oder einen Return-Button (264) aufweist, die ausgelegt sind, durch vorbestimmte Betätigungsmuster zugeordnete Funktionen der WC-Sitzgarnitur (1) zu steuern.

6. Technikbox nach einem der vorhergehenden Patentansprüche, wobei im Gestenfeld (254) Funktionen der WC-Sitzgarnitur (1) durch Verschieben eines Fingers entlang des Gestenfeldes (254) nach einem vorbestimmten Muster (touch-Geste) ausgewählt werden.

7. Technikbox nach einem der vorhergehenden Patentansprüche, wobei bei dem Bediengerät unterhalb des Gestenfelds (254) etwa mittig ein Display (258) und beidseitig davon ein OK-Button (262) und ein Return-Button (264) einerseits und eine Scrollfläche (256) andererseits angeordnet sind.

8. Technikbox nach einem der vorhergehenden Patentansprüche, wobei das Kommunikationsmodul ausgelegt ist, Wetterdaten oder sonstige, WC-Garniturunabhängige Informationen zu erfassen und an den Nutzer/die Nutzerin oder das Bediengerät weiterzugeben.

9. Technikbox nach einem der vorhergehenden Patentansprüche, wobei das Bediengerät ausgelegt ist, über die Sensorik erfasste Parameter, beispielsweise Vitalparameter, auszuwerten und die Ergebnisse der Auswertung anzuzeigen.

10. Technikbox nach einem der vorhergehenden Patentansprüche, mit einem aufladbaren Akku.

11. WC-Sitzgarnitur (1) mit einer Technikbox (2) nach einem der vorhergehenden Patentansprüche.
